# EUROPÄISCHE PATENTANMELDUNG

(11) **EP 0 900 779 A2**
(43) Veröffentlichungstag der Anmeldung: **10.03.1999**
(21) Anmeldenummer: 98115053.5
(22) Anmeldetag: 11.08.1998
(51) Int. Cl.: C07C 209/60

(54) **Einstufiges Verfahren zur Herstellung von Aminen**

(30) Priorität: 26.08.1997 DE 19737053
(71) Anmelder: Hoechst Research & Technology Deutschland GmbH & Co. KG, 65929 Frankfurt am Main (DE)
(72) Erfinder: Herwig, Jürgen, Dr., 46569 Hünxe (DE); Fischer, Richard Walter, Dr., 65812 Bad Soden (DE); Alexander, Heinz, 65929 Frankfurt (DE); Zimmermann, Burkhard, 85386 Eching (DE); Beller, Matthias, Prof. Dr., 85737 Ismaning (DE)

(57) **Zusammenfassung**

Verfahren zur Herstellung eines Amins durch einstufige Umsetzung eines C₂-C₂₀-Olefins mit einer eine NH₃-Gruppe aufweisenden Verbindung, Wasserstoff und Kohlenmonoxid, wobei die Umsetzung in Gegenwart eines Katalysators stattfindet, der als in Wasser in gelöster oder suspendierter Form vorliegt, und der Katalysator mindestens zwei Metalle aus der Gruppe VIII des Periodensystems der Elemente in elementarer oder gebundener Form enthält.

## Beschreibung

Die vorliegende Erfindung betrifft ein einstufiges Verfahren zur Herstellung von Aminen durch katalytische Umsetzung von Olefinen mit Kohlenmonoxid, Wasserstoff und einer eine NH₃-Gruppe aufweisenden Verbindung unter Druck und erhöhter Temperatur.

Amine und deren Derivate sind von industrieller Bedeutung als Vorprodukte für Farbstoffe, Feinchemikalien, Pharmazeutika, Agroprodukte und als Additive für Schmieröle und Dieselkraftstoffe. Normalerweise werden primäre Amine durch reduktive Aminierung von Aldehyden, durch Aminolyse von Alkoholen oder durch Hydrierung von Nitrilen hergestellt.

Die Aminomethylierung, also die direkte Umsetzung von Olefinen mit Ammoniak und Synthesegas, führt mit nur geringen Ausbeuten und sehr schlechten Selektivitäten zu primären oder sekundären Aminen, da diese intermediär gebildeten Produkte sehr schnell zu tertiären Aminen weiterreagieren und Nebenreaktionen wie Aldolkondensationen der intermediär gebildeten Aldehyde auftreten. In der Literatur gibt es nur sehr wenige Beispiele, die die Aminomethylierung zu Herstellung von primären Aminen beschreiben.

In US 4,794,199 wird eine Aminomethylierung mit einem phosphinmodifizierten Katalysatorsystem beschrieben, das als Katalysatormetall nur Kobalt enthält. Die Reaktionstemperaturen liegen mit 200 °C sehr hoch. Die Selektivität zu primären Aminen wird mit 57 % angegeben, davon sind aber nur etwa die Hälfte Amine, die aus der direkten Aminomethylierung stammen. Die andere Hälfte der primären Amine entstammt der Aminomethylierung des Aldolkondensationproduktes. Die eigentliche Selektivität zum gewünschten niedermolekularen primären Amin beträgt maximal 32 % in den beschriebenen Beispielen. Für eine technische Anwendung reichen die erzielten Selektivitäten nicht aus.

In Catalysis Today **36** (1997) S. 305-310 werden die Ergebnisse aus US 4,794,199 zusammengefaßt und erläutert. Der Autor kommt zu dem Schluß, daß effektivere Methoden zur einstufigen Aminherstellung aus Olefinen, Synthesegas und Ammoniak gefunden werden müssen.

Der vorliegenden Erfindung liegt die Aufgabe zugrunde, ein einstufiges Verfahren bereitzustellen, durch das Olefine, Synthesegas und Ammoniak mit hoher Selektivität zu Aminen umgesetzt werden können. Zusätzlich ist es wünschenswert, daß der Katalysator vom Produkt leicht abgetrennt werden kann.

Diese Aufgabe wird erfindungsgemäß gelöst mit einem Verfahren zur Herstellung eines Amins durch einstufige Umsetzung eines C₂-C₂₀-Olefins mit einer eine NH₃-Gruppe aufweisenden Verbindung, Wasserstoff und Kohlenmonoxid, wobei die Umsetzung in Gegenwart eines Katalysators stattfindet, der in Wasser in gelöster oder suspendierter Form vorliegt, und der Katalysator mindestens zwei Metalle aus der Gruppe VIII des Periodensystems der Elemente (Fe, Co, Ni, Ru, Rh, Rd, Os, Ir, Pt) in elementarer oder gebundener Form enthält.

Dieses Verfahren eignet sich besonders für die Herstellung primärer oder sekundärer Amine, wobei je nach Wahl der Ausgangsprodukte bevorzugt primäre oder sekundäre Amine entstehen können. Das in den anderen Fällen erhaltene Gemisch aus primären und sekundären Aminen kann durch an sich bekannte Verfahren auf destillativem oder chromatographischem Wege in die einzelnen Produkte getrennt werden.

Das erfindungsgemäße Verfahren zeichnet sich besonders dadurch aus, daß bei hohen Umsätzen in einem einstufigen Verfahren primäre oder sekundäre Amine mit jeweils hohen Selektivitäten hergestellt werden können. Tertiäre Amine entstehen in vernachlässigbaren Mengen.

Gemäß einer vorteilhaften Ausführungsform ist mindestens eines der Metalle aus der Gruppe VIII des Periodensystems der Elemente Rhodium oder Iridium. Besonders bevorzugt enthält der Katalysator sowohl Rhodium, als auch Iridium in elementarer oder gebundener Form, wobei das molare Verhältnis von Rhodium zu Iridium zwischen 2 : 1 und 1 : 200, insbesondere zwischen 1 : 1 und 1 : 100, liegt. Besonders gut eignen sich unter Reaktionsbedingungen wasserlösliche Verbindungen, die Rhodium und Iridium enthalten (z.B. in Wasser lösliche Komplexe); ebenso können die Metalle aus der Gruppe VIII des Periodensystems der Elemente in feinverteilter (kolloidaler) elementarer Form eingesetzt werden. Erfindungsgemäße verwendbare Metallverbindungen sind beispielsweise Rhodium(III)carboxylate, Rhodium(III)acetylacetonat, Rhodium(III)sulfat, Rhodium(III)nitrat, Iridium(III)acetylacetonat, Iridium(III)carboxylate, Iridium(III)sulfat, Iridium(III)nitrat und Chloro-(1,5-cyclooctadien)-iridium(I)-dimer.

Die eine NH₃-Gruppe aufweisende Verbindung kann Ammoniak oder eine Ammoniumverbindung sein.
Der Ammoniak kann der Reaktion als Gas oder in Form einer wäßrigen Lösung zugesetzt werden. Die wäßrige Lösung enthält bevorzugt 5 bis 35 Gew.-% Ammoniak bei Raumtemperatur und Normaldruck.
Die Ammoniumverbindung ist vorzugsweise eine in Wasser gut lösliche Verbindung, insbesondere ist sie ausgewählt aus der Gruppe von Ammoniumacetat, Ammoniumcarbonat, Ammoniumchlorid und Ammoniumbromid.
Sofern Ammoniak als Gas eingesetzt wird, beträgt dessen Konzentration von 0,1 bis 80 Gew.-%, vorzugsweise von 5 bis 80 Gew.-%, insbesondere von 10 bis 70 Gew.-%, bezogen auf die wäßrige Phase.
Wenn die Ammoniumverbindung als wäßrige Lösung eingesetzt wird, liegt ihre Konzentration vorzugsweise zwischen 1 und 80 Gew.-%, besonders bevorzugt zwischen 10 und 80 Gew.-%, insbesondere zwischen 20 und 40 Gew.-%, bezogen auf die wäßrige Phase.
Die Menge der eine NH₃-Gruppe aufweisenden Verbindung liegt im allgemeinen pro Mol Olefin zwischen 0,1 und 100 Mol, vorzugsweise zwischen 3 und 100 Mol. Sie liegt für eine hohe Selektivität bezüglich der primären Amine vorzugsweise zwischen 5 und 20 Mol, für eine hohe Selektivität bezüglich der sekundären Amine dagegen vorzugsweise zwischen 0,3 und 0,8 Mol.

Das Olefin kann unter Reaktionsbedingungen in einer flüssigen, mit Wasser nicht mischbaren Phase vorliegen.
Im Hinblick auf die großtechnische Anwendung dieses Verfahrens werden bevorzugt Olefine mit 3 bis 12 Kohlenstoffatomen, insbesondere mit 6 bis 10 Kohlenstoffato-men, umgesetzt.
Dabei kann das Olefin bis zu 3 nicht-konjugierte Doppelbindungen aufweisen. Ebensogut können als Olefin Cycloolefine mit bis zu 3 Carbocyclen und Arylvinylverbindungen eingesetzt werden. Es ist aber auch möglich, Mischungen der zuvor beschriebenen Olefine zu verwenden.
Besonders gut eignet sich dieses Verfahren insbesondere für die Umsetzung von Olefinen mit einer endständigen oder mit zwei nicht-konjugierten Doppelbindungen. Verbindungen mit einer endständigen Doppelbindung sind vorzugsweise Alkene, Alkylalkenoate, Alkylenalkanoate, Alkenylalkylether und Alkenole. Beispiele solcher Verbindungen sind Ethen, Propen, 1-Buten, 1-Penten, 1-Hexen, 1-Hepten, 1-Octen, 1-Decen, 1-Nonen, 1-Dodecen, 1-Hexadecen, 2-Ethyl-1-Hexen, Styrol, 3-Phenyl-1-propen, 1,4-Hexadien, 1,7-Octadien, Allylpropionat, 4-Vinylcyclohexen, Vinylnorbornen, Dicyclopentadien, Tripropylen, Dimersol, Cyclohexen, Cyclopenten, Pinen und Limonen.
Bevorzugterweise ist das Olefin ausgewählt aus der Gruppe von Propen, Buten, iso-Buten, Hepten, Hexen und Dicyclopentadien.

Gemäß einem besonders bevorzugten Verfahren entsprechend der vorliegenden Erfindung liegen der Katalysator und das Olefin in zwei verschiedenen flüssigen, miteinander nicht mischbaren Phasen vor. Darüber hinaus können die in Form von Gasen eingesetzten Edukte unter Reaktionsbedingungen in einer weiteren, gasförmigen Phase vorliegen.
Der Katalysator befindet sich in der wäßrigen Phase, während die organische Phase die Edukt- und Produktphase ist. Sie kann optional weitere inerte, mit Wasser nicht oder nur wenig mischbare Lösungsmittel enthalten. Beispiele für solche inerten Lösungsmittel sind Toluol, Benzol, Xylole, Diethylether, Methyl-tert-butylether und Alkane wie Hexan, Pentan und Oktan.
Die beiden Phasen werden üblicherweise in einem Volumenverhältnis der wäßrigen zur organischen Phase von 10 : 1 bis 1 : 10, insbesondere von 5 : 1 bis 1 : 5, eingesetzt.
Ein besonderer Vorteil hierbei besteht darin, daß die abgetrennte wäßrige Katalysatorphase wieder in die Reaktion eingesetzt werden kann. Voraussetzung hierfür ist das Vorhandensein von zwei verschiedenen flüssigen Phasen. Eventuell kann eine geringe zusätzliche Menge frischer Katalysatorlösung zugesetzt werden, um Aktivitätseinbußert auszugleichen.

Die wässerige Katalysatorphase kann des weiteren 1 bis 50 Gew.-%, insbesondere 2 bis 30 Gew.-%, Zusätze enthalten, die die katalytische Aktivität steigern. Dies können Stoffe sein, die die Lipophilität der wäßrigen Phase erhöhen, was eine höhere Wasserlöslichkeit des eingesetzten Olefins in der wässerigen Katalysatorphase unter Reaktionsbedingungen bewirkt. Solche Stoffe sind z.B. Lösungsvermittler oder Ammonium-, Alkali- oder Erdalkalisalze.

Die für die erfindungsgemäße Umsetzung verwendbaren Lösungsvermittler sind vorzugsweise ausgewählt aus der Gruppe von Mono-, Di- und Trialkoholen (beispielsweise Glycerin, Methanol oder Ethanol), Polyalkylenglykolen, Sulfolan (Tetrahydrothiophen-1,1-dioxid), N-Methylpyrrolidon, Glyme (Ethylenglycoldimethylether) und Diglyme (Diethylenglycoldimethylether).

Besonders gut eignen sich als Lösungsvermittler Polyethylenglykole der Formel (I),

R¹―(OCH₂CH₂)ₙ―OR² (I)

in der R¹ ein Wasserstoffatom, einen geradkettigen oder verzweigten Alkylrest mit 1 bis 4 Kohlenstoffatomen oder einen Hydroxyalkylrest mit 1 bis 4 Kohlenstoffatomen, insbesondere ein Wasserstoffatom, einen Methyl-, Hydroxymethyl- oder Hydroxypropylrest, darstellt; R² einen Methylrest, insbesondere ein Wasserstoffatom, darstellt; und n eine ganze Zahl zwischen 2 und 20, insbesondere zwischen 6 und 10, ist.

Beispiele für solche Verbindungen sind
- Polyethylenglykole der Formel H(OCH₂CH₂)ₙOH mit einem mittleren Molgewicht von ungefähr 200 (PEG 200), 400 (PEG 400), 600 (PEG 600) oder 1000 (PEG 1000);
- Verbindungen der Formel CH₃(OCH₂CH₂)ₙOH mit einem mittleren Molgewicht von ungefähr 350 (M 350), 500 (M 500) oder 750 (M 750);
- oder Verbindungen der Formel CH₃CHOHCH₂(OCH₂CH₂)ₙOH mit einem mittleren Molgewicht von ungefähr 300 (300 PR), 450 (450 PR), 600 (600 PR) oder 1000 (1000PR).

Bei den Polyethylenglykolen der Formel H(OCH₂CH₂)ₙOH bedeutet die Bezeichnung
- PEG 200 ein Gemisch von Polyethylenglykolen der Formel H(OCH₂CH₂)ₙOH, in der n für eine ganze Zahl von 3 bis 6 steht;
- PEG 400 ein Gemisch von Polyethylenglykolen der Formel H(OCH₂CH₂)ₙOH, in der n für eine ganze Zahl von 7 bis 10 steht;
- PEG 600 ein Gemisch von Polyethylenglykolen der Formel H(OCH₂CH₂)ₙOH, in der n für eine ganze Zahl von 11 bis 16 steht; und
- PEG 1000 ein Gemisch von Polyethylenglykolen der Formel H(OCH₂CH₂)ₙOH, in der n für eine ganze Zahl von 15 bis 30 steht.
Diesen Gemischen ist jeweils ein entsprechendes mittleres Molgewicht von etwa 200 (PEG 200), etwa 400 (PEG 400), etwa 600 (PEG 600) respektive etwa 1000 (PEG 1000) zuzuordnen.

Bei den Verbindungen der Formel CH₃(OCH₂CH₂)ₙOH bedeutet die Bezeichnung
- M 350 ein Gemisch von Verbindungen der Formel CH₃(OCH₂CH₂)ₙOH, in der n für eine ganze Zahl von 5 bis 9 steht;
- M 500 ein Gemisch von Verbindungen der Formel CH₃(OCH₂CH₂)ₙOH, in der n für eine ganze Zahl von 9 bis 13 steht; und
- M 750 ein Gemisch von Verbindungen der Formel CH₃(OCH₂CH₂)ₙOH, in der n für eine ganze Zahl von 12 bis 20 steht.
Diesen Gemischen ist jeweils ein entsprechendes mittleres Molgewicht von etwa 350 (M 350), etwa 500 (M 500) respektive 750 (M 750) zuzuordnen.

Bei den Verbindungen der Formel CH₃CHOHCH₂(OCH₂CH₂)ₙOH bedeutet die Bezeichnung
- 300 PR ein Gemisch von Verbindungen der Formel R(OCH₂CH₂)ₙOH, in der R für einen β-Hydroxypropylrest CH₃CHOHCH₂- und n für eine ganze Zahl von 6 bis 9 steht;
- 450 PR ein Gemisch von Verbindungen der Formel R(OCH₂CH₂)ₙOH, in der R für einen β-Hydroxypropylrest CH₃CHOHCH₂- und n für eine ganze Zahl von 8 bis 14 steht;
- 600 PR ein Gemisch von Verbindungen der Formel R(OCH₂CH₂)ₙOH, in der R für einen β-Hydroxypropylrest CH₃CHOHCH₂- und n für eine ganze Zahl von 12 bis 20 steht; und
- 1000 PR ein Gemisch von Verbindungen der Formel R(OCH₂CH₂)ₙOH, in der R für einen β-Hydroxypropylrest CH₃CHOHCH₂- und n für eine ganze Zahl von 18 bis 26 steht.
Diesen Gemischen ist jeweils ein entsprechendes mittleres Molgewicht von etwa 300 (300 PR), etwa 450 (450 PR), etwa 600 (600 PR) respektive etwa 1000 (1000 PR) zuzuordnen.

Besonders gute Ergebnisse werden mit einem Polyethylenglykol erhalten, das ein mittleres Molgewicht von 350 bis 450, insbesondere von ungefähr 400, aufweist. Es lassen sich auch beliebige Mischungen von verschiedenen Polyethylenglykolen, Polyethylenglykolethern (Halbether) und Polyethylenglykoldiethern verwenden.

Bei den Ammonium-, Alkali- oder Erdalkalisalzen handelt es sich vorzugsweise um solche aus der Gruppe von Natriumiodid, Kaliumiodid, Ammoniumiodid, Natriumchlorid, Kaliumchlorid, Natriumbromid und Kaliumbromid. Diese Salze steigern die katalytische Aktivität und die Stabilität des Katalysatorsystems.

In einer bevorzugten erfindungsgemäßen Ausführungsform weist der Katalysator zusätzlich mindestens einen Liganden auf, der mindestens ein Phosphoratom enthält.

Dieser Ligand ist vorteilhafterweise ein wasserlösliches organisches Phosphin, insbesondere ein sulfoniertes Triarylphosphin der Formel (II), 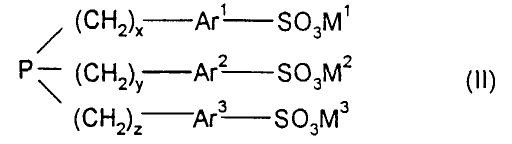 in der Ar¹, Ar² und Ar³ unabhängig voneinander jeweils einen Phenyl-, Naphthyl-, Biphenyl-, Phenylnaphthyl- oder Binaphtylrest darstellen; x, y und z unabhängig von-einander eine ganze Zahl zwischen 0 und 4, vorzugsweise zwischen 1 und 2, insbe-sondere die Zahl 1, darstellen; und M¹, M² und M³ unabhängig voneinander jeweils ein Alkalimetallion oder ein Ammoniumion darstellen. Es ist aber auch möglich, daß M¹, M² und M³ andere, höherwertige Kationen darstellen wie beispielsweise Erd-alkali- oder Zinkkationen, wobei der Ladungsausgleich maßgeblich die Anzahl dieser Kationen bestimmt.

Für das erfindungsgemäße Verfahren besonders gut bewährt haben sich trisulfonierte Triarylphosphine.
Hierbei besonders bevorzugt ist das Trinatrium-tri(m-sulfophenyl)phosphin entsprechend der Formel

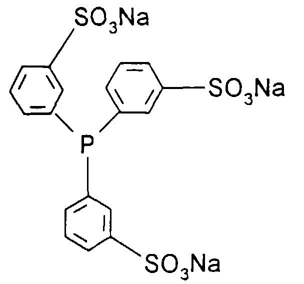

Dieses Trinatriumsalz enthält aufgrund seiner Herstellung durch Sulfonierung von Triphenylphosphin auch noch Anteile an mono- und disulfonierten Verbindungen und geringe Anteile der entsprechenden Phosphinoxide.

Das organische Phosphin kann auch ein sulfoniertes Triarylphosphin mit zwei Phosphoratomen sein, das beispielsweise einen Rest -(CH₂)ₓ-Ar-Ar-(CH₂)ₓ- enthält, worin
- x für eine ganze Zahl von 1 bis 4, insbesondere 1 bis 2, vorzugsweise 1 steht;
- Ar-Ar Biphenyl oder Binaphthyl bedeutet;
- die -(CH₂)ₓ-Gruppe mit der einen Bindung jeweils in ortho-Stellung zu der die beiden Arylreste verbindenden Aryl-Arylbindung Ar-Ar steht, und mit der anderen Bindung jeweils mit einem Phosphoratom, das jeweils zwei weitere, gleiche oder verschiedene Arylreste, insbesondere Phenylreste besitzt, verbunden ist.

Beispiele für derartige, zwei Phosphoratome enthaltende, sulfonierte Triarylphosphine sind Verbindungen der Formel (III), 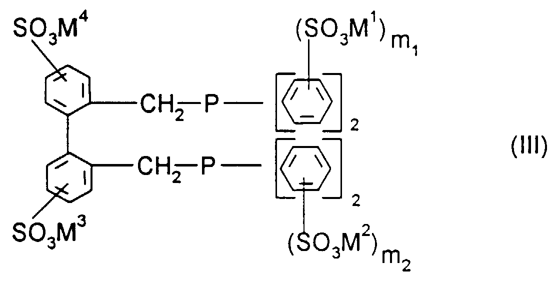 in der m¹ und m² den Wert 0 oder 1 haben können, wobei die Summe von m¹ und m² mindestens 1 beträgt; und M¹, M², M³ und M⁴ unabhängig voneinander jeweils ein Alkalimetallion oder ein Ammoniumion darstellen. Auch hier können M¹, M² und M³ andere, höherwertige Kationen darstellen wie beispielsweise Erdalkali- oder Zinkkationen, wobei der Ladungsausgleich wiederum maßgeblich die Anzahl dieser Kationen bestimmt.

Das zwei Phosphoratome enthaltende, sulfonierte Triarylphosphin kann aber auch eine Verbindung der Formel (IV) 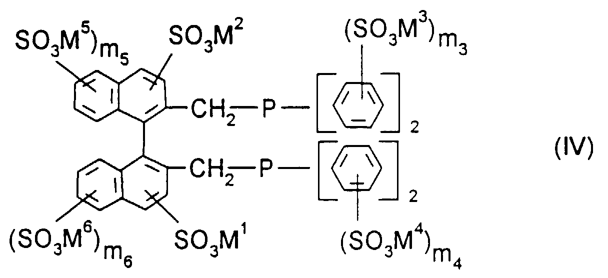 sein, in der m₃, m₄, m₅ und m₆ den Wert 0 oder 1 haben können, wobei die Summe von m₃, m₄, m₅ und m₆ mindestens 2 beträgt; und M¹, M², M³, M⁴, M⁵ und M⁶ unabhängig voneinander die gleiche Bedeutung wie im vorangegangenen haben.
Solche zwei Phosphoratome enthaltenden Triarylphosphine weisen mindestens drei -SO₃M Reste, insbesondere vier bis acht -SO₃M Reste, auf, worin M die gleiche Bedeutung besitzt wie M¹ bis M⁶. Die -SO₃M Reste sitzen üblicherweise an den Arylresten des Restes -(CH₂)ₓ-Ar-Ar-(CH₂)ₓ- und an den zwei weiteren Arylresten, die mit dem Phosphor verbunden sind.
Alternativ können anstelle sulfonierter Triarylphosphine andere Triarylphosphine als Liganden eingesetzt werden, bei denen die SO₃M-Gruppe durch andere Gruppen ersetzt wird, die eine Wasserlöslichkeit des Triarylphosphines bewirken, wie z.B. PO₃M₂-Gruppen.

Die wäßrige Phase kann 5 bis 2.000 ppm an Metallen aus der Gruppe VIII des Periodensystems der Elemente enthalten.
Vorzugsweise wird die wäßrige, den Katalysator enthaltende Phase entsprechend einer Menge von 2^{x}10⁻⁶ bis 5^{x}10⁻² Mol der Metalle aus der Gruppe VIII des Periodensystems der Elemente pro Mol olefinischer Verbindung eingesetzt.
Das Verhältnis der Metalle aus der Gruppe VIII des Periodensystems der Elemente zum Liganden kann zwischen 1 : 2 und 1 : 500, vorzugsweise zwischen 1 : 5 und 1 : 200, insbesondere zwischen 1 : 10 und 1 : 100, liegen.

Das Verhältnis von Kohlenmonoxid und Wasserstoff kann in weiten Grenzen variieren. Günstig ist ein Verhältnis von Kohlenmonoxid zu Wasserstoff von 10 : 1 bis 1 : 30, insbesondere von 5:1 bis 1:8, besonders bevorzugt von 1:2 bis 1:5. Ebenfalls vorteilhaft ist ein Zupressen von Synthesegas im Verhältnis Kohlenmonoxid zu Was-serstoff von 1:1 bis 1:5, insbesondere von 1 : 1 bis 1:3 und gegebenenfalls späteres Zupressen von reinem Wasserstoff im Verlauf der Reaktion.
Kohlenmonoxid und Wasserstoff können mit einem Druck von 5 bis 30 MPa, vorzugsweise von 10 bis 18 MPa, zugesetzt werden.

Die Temperatur liegt während der Reaktion im allgemeinen zwischen 60 und 200 °C, vorzugsweise zwischen 110 und 160 °C, insbesondere zwischen 135 und 150 °C.
Als Reaktionsgefäße werden vorzugsweise Druckreaktoren mit magnetischer oder mechanischer Rühr- oder Mischeinrichtung verwendet. Während der Umsetzung ist eine gute Durchmischung der vorhandenen Phasen, d.h. von wässeriger Phase, Kohlen-monoxid/Wasserstoff und ggf. organischer Phase sicherzustellen. Dies kann insbe-sondere durch intensives Rühren und/oder Umpumpen von organischer und wässriger Phase bewirkt werden.

Die Reaktionsführung erfolgt beispielsweise so, daß die wäßrige Ammoniaklösung und der Ligand mit den Rhodium- oder/und Iridiumverbindungen und eventuellen Zusätzen vorgelegt wird und erst dann Kohlenmonoxid und Wasserstoff eingeleitet werden. Dieses Gemisch kann 0,5 - 3 Stunden bei Reaktionstemperatur präformiert werden, und dann können das Olefin und optional das oder die inerten Lösungsmittel mit Hilfe einer Pumpe zudosiert werden.
Die Reaktionszeit beträgt 1 - 50, vorzugsweise 3 - 20 Stunden. Eine kontinuierliche Führung des Versuches ist ebenfalls möglich.

Nach dem Ende der Reaktion wird der Druckreaktor gekühlt, durch Druckentspannung von Kohlenmonoxid und Wasserstoff befreit und das Reaktionsgemisch entnommen. Bei Abschalten der Durchmischungseinrichtung trennen sich die Phasen von allein. Die organische Phase kann destillativ aufgearbeitet werden und kann dann bei Bedarf gaschromatographisch untersucht werden.

Die folgenden Beispiele dienen der Erläuterung der Erfindung.

### Beispiel 1 (Vergleichsbeispiel):

In einem 200 ml Autoklav mit intensiver magnetischer Rührung werden 5,2 mg Rhodiumacetat, 40 ml 33 %ige Ammoniaklösung und 10 ml einer 0,5 molaren wäßrigen Lösung des Natriumsalzes der Triphenylphosphan-m-trisulfonsäure gegeben. Als organische Phase werden 40 ml Ether und 17.4 g 1-Hexen hinzugegeben. Es werden 100 bar Wasserstoff und 50 bar Kohlenmonoxid aufgedrückt, und es wird eine Temperatur von 140 °C eingestellt. Der verbrauchte Wasserstoff und das verbrauchte Kohlenmonoxid werden durch Nachpressen derselben ersetzt.
Nach 6 Stunden wird der Autoklav abgekühlt und entspannt, die organische Phase entnommen und gaschromatographisch untersucht.
Der Umsatz beträgt 19,2 % und die Selektivität zu primären Heptylaminen 0 %. Der Anteil von Iminen (isomeren Iminen gebildet aus der Kondensation von intermediär gebildeten Aldehyden und primären Aminen) im Gemisch beträgt 7,6 %, der der sekundären Amine 0 %.

### Beispiel 2:

In einem 200 ml Autoklav mit intensiver magnetischer Rührung werden 5,2 mg Rhodiumacetat, 100 mg Chloro(1,5-cyclooctadien)-iridium(I)-dimer, 40 ml 33 %ige Ammoniaklösung und 10 ml einer 0,5 molaren Lösung des Natriumsalzes der Triphenylphosphin-m-trisulfonsäure gegeben. Als organische Phase werden 30 ml Toluol und 5,8 g 1-Hexen hinzugegeben. Es werden 100 bar Wasserstoff und 50 bar Kohlenmonoxid aufgedrückt und eine Temperatur von 140 °C eingestellt. Nach 6 Stunden wird der Autoklav abgekühlt und entspannt, die organische Phase entnom-men und gaschromatographisch untersucht.
Bei einem Umsatz von 54 % wird eine Selektivität von 60 % zu primären Heptylaminen festgestellt. Der Anteil von Iminen (isomeren Iminen gebildet aus der Kondensation von intermediär gebildeten Aldehyden und primären Aminen) im Gemisch beträgt 12 %, der der sekundären Amine 1,7 %.

### Beispiele 3 bis 11:

Diese Beispiele werden analog zu Beispiel 2 durchgeführt. Die Edukte und deren Mengen sowie die Reaktionsergebnisse sind in Tabelle 1 angegeben. Bei den Beispielen 3 bis 8 und 10 bis 12 wurden zwei flüssige Phasen beobachtet, beim Beispiel 9 hingegen nur eine Phase.

In der Tabelle 1 werden folgende Bezeichnungen verwendet:
- Rh steht für Rhodiumacetat;
- Ir steht für Chloro(1,5-cyclooctadien)-iridium(I)-dimer;
- H₂ steht für Wasserstoff;
- CO steht für Kohlenmonoxid;
- NH₃ steht für eine 33 %ige Ammoniaklösung; lediglich in Beispiel 12 steht NH₃ für Ammoniumacetat, die Menge bezieht sich hierbei auf Gramm;
- TPPTS steht für Trinatrium-tri(m-sulfophenyl)phosphin;
- PEG 400 steht für ein Polyethylenglykol mit einem mittleren Molgewicht von 400;
- LM steht für den Begriff Lösungsmittel; Ether steht hierbei für Diethylether;
- t bezeichnet die Reaktionsdauer;
- die Selektivität bezogen auf die Imine bezieht sich auf isomere Imine, die aus der Kondensation von intermediär gebildeten Aldehyden und primären Aminen entstehen;
- THF steht für Tetrahydrofuran.

### Beispiel 12:

In einem 200 ml Autoklav mit intensiver magnetischer Rührung werden 5,2 mg Rhodiumacetat, 100 mg Chloro(1,5-cyclooctadiene)-iridium(I)-dimer, 40 ml demineralisiertes Wasser, 30 g Ammoniumacetat und 10 ml einer 0,5 molaren Lösung des Natriumsalzes der Triphenylphosphan-m-trisulfonsäure gegeben. Als organische Phase werden 40 ml Diethylether und 5,8 g 1-Hexen hinzugegeben. Es werden 125 bar Wasserstoff und 25 bar Kohlenmonoxid aufgedrückt und eine Temperatur von 140 °C eingestellt. Nach 360 Minuten wird der Autoklav abgekühlt und entspannt, die wäßrige Phase mit NaOH auf einen pH-Wert von 14 gebracht, die organische Phase entnommen und gaschromatographisch untersucht. Bei einem Umsatz von 90,7 % wird eine Selektivität von 56 % zu primären Heptylaminen festgestellt. Der Anteil von Iminen (isomeren Iminen gebildet aus der Kondensation von intermediär gebildeten Aldehyden und primären Aminen) im Gemisch beträgt 0 %, der der sekundären Amine 9,4 %.

### Beispiel 13:

In einen 200 ml Autoklav mit intensiver magnetischer Rührung werden 26 mg Rhodiumacetat, 100 mg Chloro(1,5-cyclooctadien)-iridium(I)-dimer, 30 ml Ammoniaklösung 33 %, 10 ml demineralisiertes Wasser und 10 ml einer 0,5 molaren Lösung des Natriumsalzes der Triphenylphosphan-m-trisulfonsäure gegeben.
Als organische Phase werden 40 ml Diethylether und 8,5 g 1-Propen hinzugegeben. Es werden 50 bar Synthesegas (CO/H₂) und 50 bar Wasserstoff aufgedrückt, und es wird einen Innentemperatur von 145°C eingestellt. Innerhalb der Versuchsdauer von 360 Minuten wird Synthesegas/Wasserstoff nachgedrückt. Das Verhältnis CO/H₂ soll 1 : 2 sein. Nach Versuchsende wird der Autoklav abgekühlt und entspannt, die wässrige von der organischen Phase getrennt.
Von der organischen Phase wird eine Probe entnommen und quantitativ gaschromatographisch untersucht. Die Ausbeute der entstandenen Amine betrug 61,2 %, und die Selektivität betrug 84,5 % (siehe Tabelle 2).

### Beispiele 14 bis 22:

In gleichartiger Weise wie in Beispiel 13 beschrieben wurden die Beispiele 14 bis 22 ausgeführt. Die geänderten Bedingungen und die Ergebnisse sind in Tabelle 2 enthalten. Die Angabe 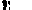 6,4 g NH₃" in Beispiel 18 bedeutet, daß zusätzlich noch 6,4 g NH₃ als Gas in den Autoklaven gegeben werden; dies wird technisch so durchgeführt, daß die 30 %ige Ammoniak-Lösung abgekühlt wird, wobei sich die Löslichkeit für Ammoniak erhöht, und daß dann Ammoniak-Gas in die abgekühlte Lösung gegeben wird, bis sich die Menge 6,4 g darin befindet.

### Beispiel 23:

In einen 200 ml-Autoklav mit intensiver magnetischer Rührung werden 2.3 mg Chloro(1,5-cyclooctadien)-rhodium(I)-dimer, 50 mg Chloro(1,5-cyclooctadien)-iridium(I)-dimer, 20 ml 33 %-ige Ammoniaklösung und 9 g einer BINAS-Lösung (139 mmol P(III) pro kg) gegeben. Als organische Phase werden 18 ml MTBE, 2.5 ml Isooctan (interner GC-Standard) und 2.5 g 1-Penten hinzugegeben. Es werden 13 bar Kohlenmonoxid und 65 bar Wasserstoff aufgedrückt, und anschließend wird eine Temperatur von 130 °C eingestellt.
Nach 12 Stunden wird der Autoklav abgekühlt und entspannt, die organische Phase entnommen und die wäßrige Phase zweimal mit je 10 ml MTBE nachextrahiert. Die vereinigten organischen Phasen werden gaschromatographisch untersucht. Der Umsatz beträgt 42 %, die Selektivität bezüglich des sekundären Amins (Di-(n-hexyl)-amin) beträgt 16,2 %, das primäre Amin wird zu 59,7 % gebildet, und das Verhältnis von linearen zu verzweigten Produkten beträgt 99:1.

### Beispiele 24 bis 26:

Diese Beispiele werden analog zu Beispiel 23 durchgeführt. Die Edukte und deren Mengen sowie die Reaktionsergebnisse sind in Tabelle 3 angegeben. In den Beispielen 25 und 26 werden jeweils 19 ml Wasser zur Ergänzung der wäßrigen Phase zugegeben. Vor der Extraktion der wäßrigen Phase nach der Reaktion werden in den Beispielen 25 und 26 je 10 ml einer 33 %-igen Ammoniak-Lösung zugegeben.

In Tabelle 3 werden folgende Bezeichnungen verwendet:
- Rh steht für Chloro(1,5-cyclooctadien)-rhodium(I)-dimer;
- Ir steht für Chloro(1,5-cyclooctadien)-iridium(I)-dimer
- H₂ steht für Wasserstoff
- CO steht für Kohlenmonoxid
- NH₃ steht für eine 33 %-ige Ammoniaklösung
- BINAS steht für eine wäßrige Lösung des Octanatriumsalzes des 8-fach sulfonierten Liganden 1,1'-Bisnaphthalin-2,2'-diylbis(methylen)bis(diphenylphosphan) (NAPHOS);
- LM steht für den Begriff Lösungsmittel, Ether steht für Diethylether, MTBE steht für Methyl-tert.-butylether;
- t steht für die Reaktionsdauer;
- n/i bedeutet das Verhältnis von linearem zu verzweigtem Produkt bei den primären Aminen.

### Beispiele 27 bis 30:

Diese Beispiele werden analog zu Beispiel 23 durchgeführt. Die Edukte und deren Mengen sowie die Reaktionsergebnisse sind in Tabelle 4 angegeben. In den Beispielen 27 bis 30 werden jeweils 19 ml Wasser zur Ergänzung der wäßrigen Phase zugegeben. Vor der Extraktion der wäßrigen Phase nach der Reaktion werden in diesen Beispielen je 10 ml einer 33 %-igen Ammoniak-Lösung zugegeben. Beispiel 30 ist ein Vergleichsbeispiel ohne Einsatz von Iridium.

In Tabelle 2 werden die gleichen Bezeichnungen wie in Tabelle 1 verwendet.

In Tabelle 4 werden die gleichen Bezeichnungen wie in Tabelle 1 bzw. 2 verwendet.

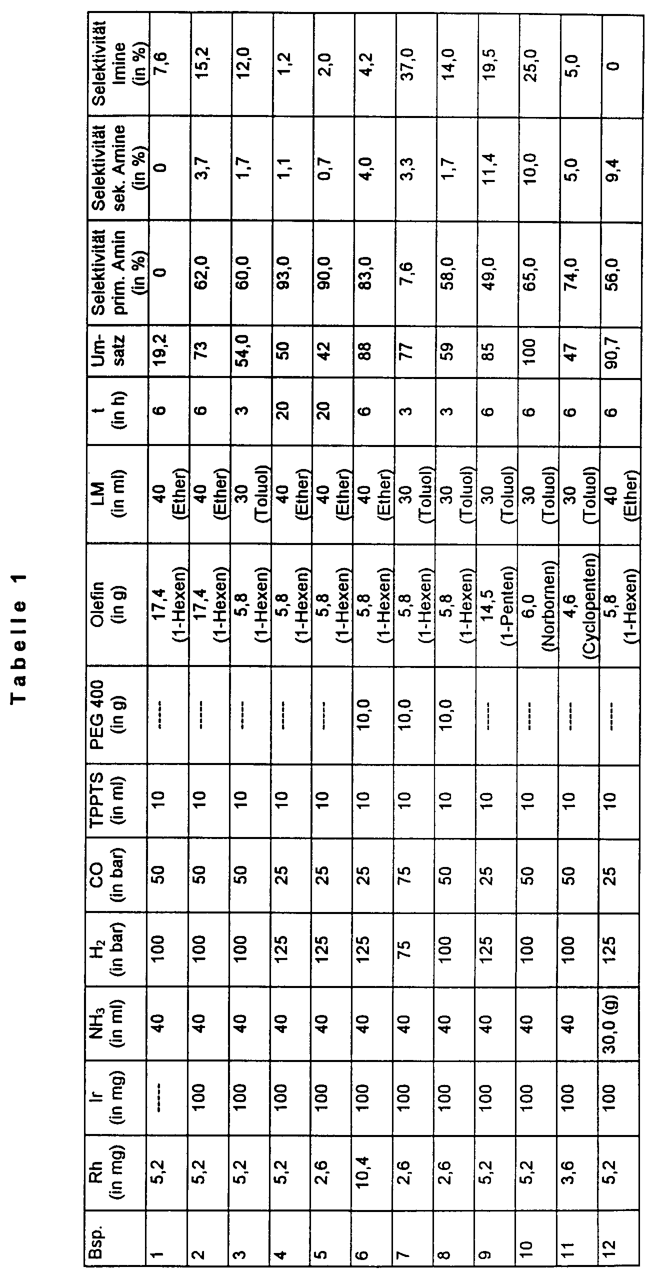

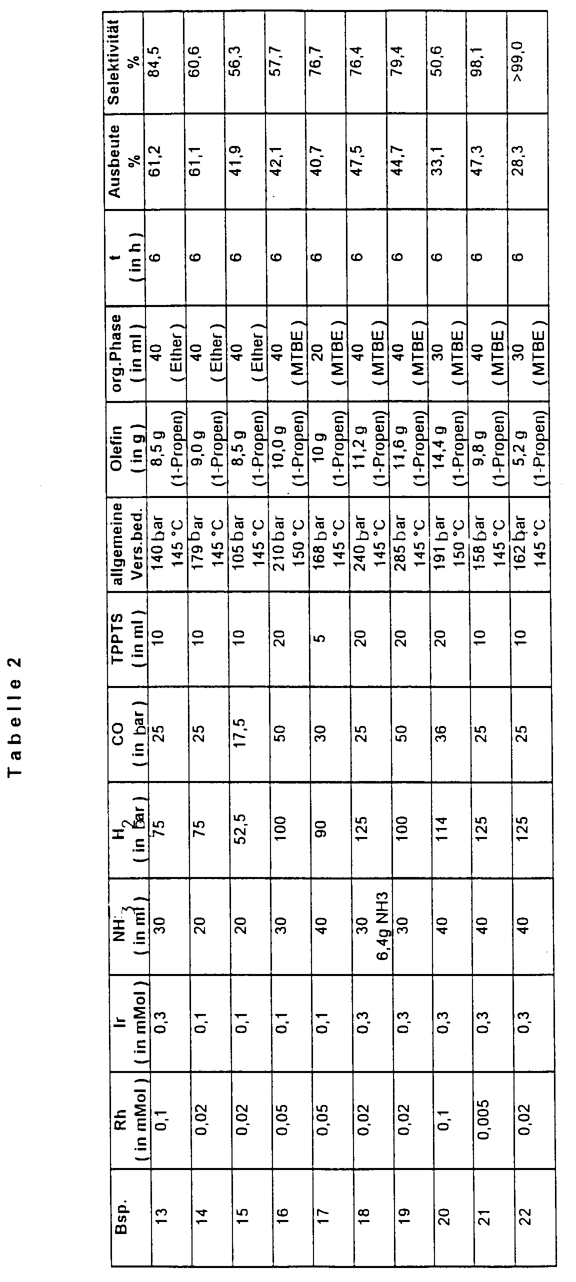

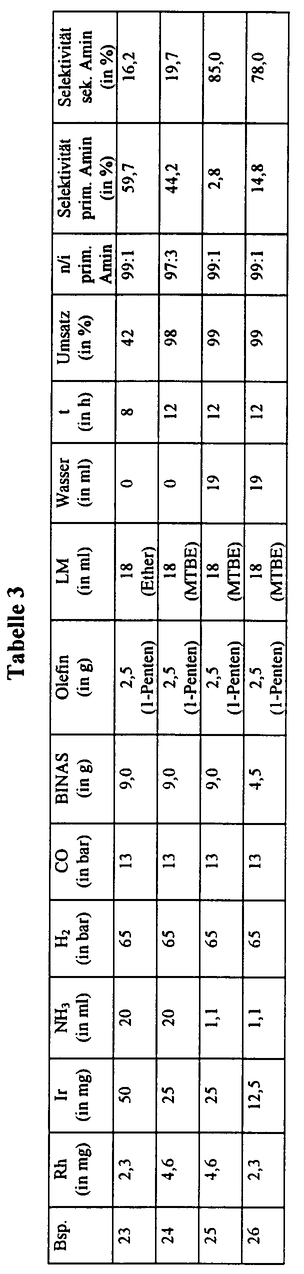

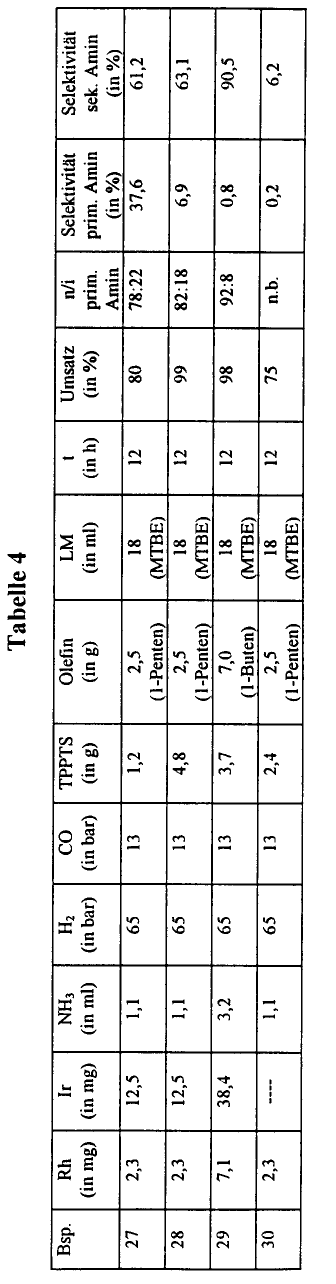

## Patentansprüche

1. Verfahren zur Herstellung eines Amins durch einstufige Umsetzung eines C₂-C₂₀-Olefins mit einer eine NH₃-Gruppe aufweisenden Verbindung, Wasserstoff und Kohlenmonoxid, dadurch gekennzeichnet, daß
(a) die Umsetzung in Gegenwart eines Katalysators stattfindet, der in Wasser in gelöster oder suspendierter Form vorliegt; und
(b) der Katalysator mindestens zwei Metalle aus der Gruppe VIII des Periodensystems der Elemente in elementarer oder gebundener Form enthält.

2. Verfahren nach Anspruch 1, dadurch gekennzeichnet, daß das Amin ein primäres oder sekundäres Amin ist.

3. Verfahren nach Anspruch 1 oder 2, dadurch gekennzeichnet, daß mindestens eines der Metalle aus der Gruppe VIII des Periodensystems der Elemente Rhodium oder Iridium ist.

4. Verfahren nach einem der vorherigen Ansprüche, dadurch gekennzeichnet, daß der Katalysator sowohl Rhodium als auch Iridium in elementarer oder gebundener Form enthält, wobei das molare Verhältnis von Rhodium zu Iridium zwischen 2 : 1 und 1 : 200, insbesondere zwischen 1 : 1 und 1 : 100, liegt.

5. Verfahren nach einem der vorherigen Ansprüche, dadurch gekennzeichnet, daß die eine NH₃-Gruppe aufweisenden Verbindung Ammoniak oder eine Ammoniumverbindung ist.

6. Verfahren nach Anspruch 5, dadurch gekennzeichnet, daß Ammoniak als Gas oder als in Wasser gelöstes Gas eingesetzt wird, vorzugsweise in einer Konzentration von 0,1 bis 80 Gew.-%, besonders bevorzugt von 5 bis 80 Gew.-%, bezogen auf die wäßrige Phase.

7. Verfahren nach Anspruch 5, dadurch gekennzeichnet, daß die Ammoniumverbindung als wäßrige Lösung eingesetzt wird, vorzugsweise in einer Konzentration von 1 bis 80 Gew.-%, besonders bevorzugt von 10 bis 80 Gew.-%, insbesondere in einer Konzentration von 20 bis 40 Gew.-%, bezogen auf die wäßrige Phase.

8. Verfahren nach einem der vorherigen Ansprüche, dadurch gekennzeichnet daß pro Mol Olefin 0,1 bis 100 Mol der eine NH₃-Gruppe aufweisenden Verbindung eingesetzt werden, vorzugsweise 3 bis 100 Mol, besonders bevorzugt 0,3 bis 0,8 Mol (für sekundäre Amine) bzw. 5 bis 20 Mol (für primäre Amine).

9. Verfahren nach einem der vorherigen Ansprüche, dadurch gekennzeichnet daß das Olefin unter Reaktionsbedingungen in einer flüssigen, mit Wasser nicht mischbaren Phase vorliegt.

10. Verfahren nach einem der vorherigen Ansprüche, dadurch gekennzeichnet, daß das Olefin 3 bis 12 Kohlenstoffatome aufweist.

11. Verfahren nach einem der vorherigen Ansprüche, dadurch gekennzeichnet, daß das Olefin ausgewählt ist aus Olefinen mit bis zu 3 nicht-konjugierten Doppelbindungen, Cycloolefinen mit bis zu 3 Carbocyclen, und Arylvinylverbindungen oder deren Mischungen.

12. Verfahren nach einem der vorherigen Ansprüche, dadurch gekennzeichnet daß das Olefin eine endständige oder zwei nicht-konjugierten Doppelbindungen aufweist.

13. Verfahren nach Anspruch 12, dadurch gekennzeichnet, daß das Olefin ausgewählt ist aus der Gruppe von Propen, Buten, iso-Buten, Hepten, Hexen und Dicyclopentadien.

14. Verfahren nach einem der vorherigen Ansprüche, dadurch gekennzeichnet, daß die Umsetzung in Gegenwart mindestens eines zusätzlichen Lösungsvermittlers durchgeführt wird, der vorzugsweise ausgewählt ist aus der Gruppe der Mono-, Di- und Trialkoholen, Polyalkylenglykolen, Sulfolan, N-Methylpyrrolidon, Glyme und Diglyme.

15. Verfahren nach Anspruch 14, dadurch gekennzeichnet, daß der Lösungsvermittler ein Polyethylenglykol der Formel (I)
R¹―(OCH₂CH₂)ₙ―OR² (I)
ist, in der
• R¹ ein Wasserstoffatom, einen geradkettigen oder verzweigten Alkylrest mit 1 bis 4 Kohlenstoffatomen oder einen Hydroxyalkylrest mit 1 bis 4 Kohlenstoffatomen, insbesondere ein Wasserstoffatom, einen Methyl-, Hydroxymethyl- oder Hydroxypropylrest, darstellt;
• R² einen Methylrest, insbesondere ein Wasserstoffatom, darstellt;
• n eine ganze Zahl zwischen 2 und 20, insbesondere zwischen 6 und 10, darstellt.

16. Verfahren nach Anspruch 15, dadurch gekennzeichnet, daß das Polyethylenglykol ein mittleres Molgewicht von 350 bis 450, insbesondere von ungefähr 400, aufweist.

17. Verfahren nach einem der vorherigen Ansprüche, dadurch gekennzeichnet, daß die Umsetzung in Gegenwart eines zusätzlichen Ammonium-, Alkali- oder Erdalkalisalzes durchgeführt wird, das vorzugsweise ausgewählt ist aus der Gruppe von Natriumiodid, Kaliumiodid, Ammoniumiodid, Natriumchlorid, Kaliumchlorid, Natriumbromid und Kaliumbromid.

18. Verfahren nach einem der vorherigen Ansprüche, dadurch gekennzeichnet, daß der Katalysator zusätzlich mindestens einen Liganden enthält, der mindestens ein Phosphoratom aufweist.

19. Verfahren nach einem der vorherigen Ansprüche, dadurch gekennzeichnet, daß der Ligand ein wasserlösliches organisches Phosphin ist.

20. Verfahren nach Anspruch 19, dadurch gekennzeichnet, daß das wasserlösliche organische Phosphin ein sulfoniertes Triarylphosphin der Formel (II) 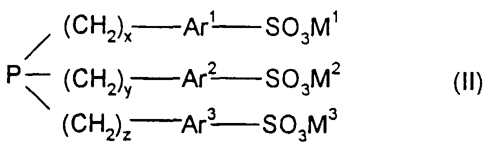 ist, in der
• Ar¹, Ar² und Ar³ unabhängig voneinander jeweils einen Phenyl-, Naphtyl-, Biphenyl-, Phenylnaphtyl- oder Binaphtylrest darstellen;
• x, y und z unabhängig voneinander eine ganze Zahl zwischen 0 und 4, vorzugsweise zwischen 1 und 2, insbesondere die Zahl 1, darstellen; und
• M¹, M² und M³ unabhängig voneinander jeweils ein Alkalimetallion oder ein Ammoniumion darstellen.

21. Verfahren nach Anspruch 20, dadurch gekennzeichnet, daß das sulfonierte Triarylphosphin ein trisulfoniertes Triarylphosphin ist, insbesondere Trinatrium-tri(m-sulfophenyl)phosphin.

22. Verfahren nach Anspruch 19, dadurch gekennzeichnet, daß das organische Phosphin eine Verbindung der Formel (III) 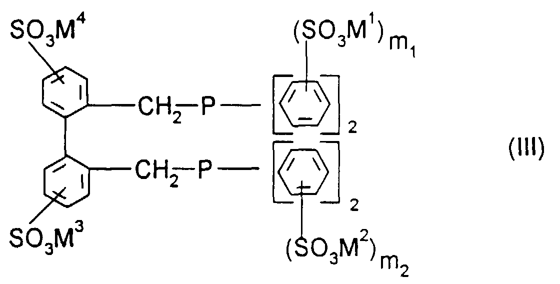 ist, in der
• m¹ und m² den Wert 0 oder 1 haben können, wobei die Summe von m¹ und m² mindestens 1 beträgt; und
• M¹, M², M³ und M⁴ unabhängig voneinander jeweils ein Alkalimetallion oder ein Ammoniumion darstellen.

23. Verfahren nach Anspruch 19, dadurch gekennzeichnet, daß das organische Phosphin eine Verbindung der Formel (IV) 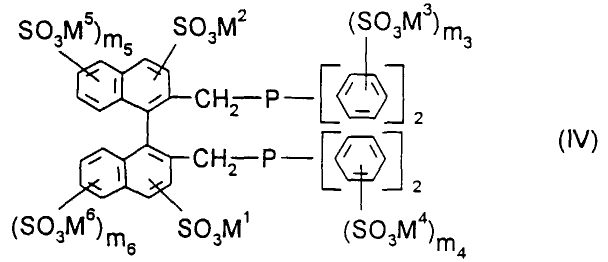 ist, in der
• m₃, m₄, m₅ und m₆ den Wert 0 oder 1 haben können, wobei die Summe von m₃, m₄, m₅ und m₆ mindestens 2 beträgt; und
• M¹, M², M³, M⁴, M⁵ und M⁶ unabhängig voneinander jeweils ein Alkalimetallion oder ein Ammoniumion darstellen.

24. Verfahren nach einem der vorherigen Ansprüche, dadurch gekennzeichnet, daß die wäßrige Phase 5 bis 2.000 ppm an Metallen aus der Gruppe VIII des Periodensystems der Elemente enthält.

25. Verfahren nach einem der vorherigen Ansprüche, dadurch gekennzeichnet daß die wäßrige, den Katalysator enthaltende Phase entsprechend einer Menge von 2^{x}10⁻⁶ bis 5^{x}10⁻² Mol der Metalle aus der Gruppe VIII des Periodensystems der Elemente pro Mol olefinischer Verbindung eingesetzt wird.

26. Verfahren nach einem der vorherigen Ansprüche, dadurch gekennzeichnet, daß das Verhältnis der Metalle aus der Gruppe VIII des Periodensystems der Elemente zum Liganden zwischen 1 : 2 und 1 : 500, vorzugsweise zwischen 1 : 5 und 1 : 200, insbesondere zwischen 1 : 10 und 1 : 100, liegt.

27. Verfahren nach einem der vorherigen Ansprüche, dadurch gekennzeichnet, daß Kohlenmonoxid und Wasserstoff mit einem Druck von 5 bis 30 MPa, vorzugsweise von 10 bis 18 MPa, zugesetzt werden, wobei insbesondere das Verhältnis von Kohlenmonoxid zu Wasserstoff zwischen 10 : 1 und 1 : 30 liegt.

28. Verfahren nach einem der vorherigen Ansprüche, dadurch gekennzeichnet, daß die Temperatur während der Reaktion zwischen 60 und 200 °C, vorzugsweise zwischen 110 und 160 °C, insbesondere zwischen 135 und 150 °C, liegt.
